(19) **Europäisches Patentamt / European Patent Office / Office européen des brevets**

(11) **EP 4 301 227 B1**

(12) **EUROPEAN PATENT SPECIFICATION**

(45) Date of publication and mention
of the grant of the patent:
**16.04.2025 Bulletin 2025/16**

(21) Application number: **22709754.0**

(22) Date of filing: **04.03.2022**

(51) International Patent Classification (IPC):
**A61B 5/22** *(2006.01)*     **A61B 5/087** *(2006.01)*
**A61B 5/00** *(2006.01)*     **A63B 23/18** *(2006.01)*

(52) Cooperative Patent Classification (CPC):
**A61B 5/224; A61B 5/087; A61B 5/6898;**
**A63B 21/00069; A63B 23/18;** A61B 2505/09;
A63B 2071/063; A63B 2220/56; A63B 2220/62;
A63B 2220/64; A63B 2225/50

(86) International application number:
**PCT/EP2022/055595**

(87) International publication number:
**WO 2022/184908 (09.09.2022 Gazette 2022/36)**

(54) **METHODS AND SYSTEMS FOR RESPIRATORY TRAINING AND TESTING**

VERFAHREN UND SYSTEME ZUM TRAINIEREN UND TESTEN DER ATMUNG

PROCÉDÉS ET SYSTÈMES D'ENTRAÎNEMENT ET DE TEST RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **04.03.2021  EP 21160622**

(43) Date of publication of application:
**10.01.2024  Bulletin 2024/02**

(73) Proprietor: **Airofit A/S**
**2200 København N (DK)**

(72) Inventors:
• **POULSEN, Christian Tullberg**
**2100 Købenahvn (DK)**
• **MCCONNELL, Alison**
**2100 København (DK)**

(74) Representative: **Høiberg P/S**
**Adelgade 12**
**1304 Copenhagen K (DK)**

(56) References cited:
**DE-A1- 102019 001 926     US-A1- 2009 229 611
US-A1- 2019 299 055        US-A1- 2021 001 169**

## Description

The present disclosure relates to and systems and computer implemented methods for respiratory training and testing. A system is configured for measuring a respiratory pressure of a subject in a mouthpiece, transmit pressure data to a remote processing device and continuously process said pressure data for calculation of respiratory training intensity and respiratory muscle performance.

## Background

Respiratory training can be defined as performing specific exercises that aim to improve the function of the respiratory muscles, i.e. the muscles involved in active inspiration and/or active expiration. Respiratory training covers both inspiratory training and expiratory training and has been shown to offer a wide range of beneficial effects. For example, inspiratory training has been shown to improve respiratory muscle function in terms of strength, power, endurance and efficiency and has been proven to be important for reducing dyspnoea on exertion.

Respiratory exercisers are known in the art and typically comprise a mouthpiece for inhalation and exhalation, and a respiratory pathway with an aperture. The aperture ensures that the subject experiences a pressure load during respiration, and thereby a respiratory muscle training stimulus. The aperture may be adjustable so that the pressure load can be set depending on the subject's respiratory strength and the type of training benefits desired.

In US Pat. No. US 4,221,381, the adjustment structure is regulating the size of at least one of the openings therein to control the size of the access opening to the atmosphere. The air resistance is the same for both inhalation and exhalation.

In US Pat. No. US 4,739,987, two openings regulate the inhalation and exhalation resistance, by the use of an aperture in each of two partitions that cover the openings to be brought into and out of alignment.

DE 10 2019 001926 discloses a device, a process and a computer program for influencing the inspiratory muscles of a person, especially but not exclusively to a concept for training the inspiratory muscles of a training person or of a patient based on electromyographic signals of his/her inspiratory muscles. The EMG measurements of the inspiratory muscles are optionally combined with pneumatic signals to determine the time curve of breathing performance and work of breathing.

US 2009/229611 discloses to a system and a method for inspiratory muscle strength training in patients receiving mechanical ventilation support.

These prior art devices and systems are associated with a number of inconveniences and problems that may be overcome by the presently disclosed methods and systems. In particular the known devices are limited in relation to the feedback that is given to the user, in relation to the control of the resistance, in relation to the mechanical implementation of the device and in relation to measurement of important training-related variables, such as pressure.

## Summary

The present invention is defined in the appended claims.

The present inventors have identified that the pressure load, i.e. the training stimulus magnitude, created by a given aperture is ostensibly proportional to the rate of airflow through the aperture, such that at a given flow rate, a smaller surface area creates a larger pressure load. Thereby, the same pressure load can be created by apertures with differing surface areas by means of varying the rate of airflow. This is a limitation of respiratory training units of the prior art, since changes to airflow rate result in changes in pressure/training load.

Faced with the drawbacks of respiratory training units of the prior art, the present inventors have realized how respiratory training units can be configured for obtaining more accurate respiratory training information, and how to provide said training information to a subject, thereby allowing the subject to optimize the training stimulus, instantaneously and/or between training sessions. Furthermore, unlike parameters for assessing respiratory training of the prior art, such as the above mentioned flow rate and pressure load, the present inventors have also realized that basing the determination of respiratory training stimulus on respiratory muscle power and/or respiratory muscle work are far superior as these parameters are both multidimensional, providing the most accurate quantification of the training stimulus.

The present disclosure therefore, in a first aspect, relates to a computer implemented method for calculation of respiratory training intensity in a dataset representing a subject breathing through separate inhalation and exhalation airways having predefined respiratory resistances, the method comprising the steps of:

- calculating the respiratory muscle power vs. time of at least one breath of the subject, and/or
- calculating the respiratory muscle work of at least one breath of the subject.

According to the claimed invention, the calculation of individual subject's respiratory training intensity is a real-time process carried out using a stream of data continuously received, such as from a pressure sensor located in a mouthpiece of a respiratory training unit. The method may be applied to calculation of inspiratory muscle training intensity and/or expiratory muscle training intensity, depending on the desired type of training.

Real-time calculation of continuously received data allows for continuous and instantaneous optimization of the training stimulus, and thereby optimization of the physiological effect of a training session. The data may be a dataset comprising pressure data vs. time acquired in connection with at least one of an inhalation and exhalation airways. The dataset may for example have been obtained by a pressure sensor for measuring air pressure in a mouthpiece.

The training stimulus may be considered to be a result of overloading the respiratory muscles, in terms of training duration, intensity and/or frequency, and further a result of the specificity of the training, e.g. strength training stimuli or endurance-training stimuli.

According to the claimed invention, the method is configured to calculate the respiratory muscles' peak respiratory muscle power output of at least one breath of the subject and/or to calculate the average peak respiratory muscle power of a plurality of breaths of the subject. It has been identified that the pressure load that elicits the respiratory muscles' peak respiratory muscle power output induces the largest and widest range of adaptations (most versatile stimulus). It should be noted that each individual has a unique peak respiratory muscle power output, for example a maximum inspiratory and/or expiratory power output, which is defined by the strength of their respiratory muscles and their ability to generate airflow, specifically, their maximal respiratory pressures (inspiratory and expiratory) and maximum respiratory flow rates (inspiratory and expiratory), respectively.

In a further aspect, the present disclosure relates to a respiratory sensing system arranged / suitable for being connected to a respiratory training or testing device, typically having an inspiratory airway and an expiratory airway. The sensing system comprising:

    a. an electronic sensor unit comprising;

        i. at least one pressure sensor for measuring air pressure in the inspiratory airway and/or the expiratory airway of the respiratory training and testing device, and
        ii. a processing unit for transmitting the pressure data, and

    b. a computer program having instructions, which, when executed by a remote processing device, such as a smartphone, cause the processing device to:

        i. continuously obtaining said pressure data via a receiver on the remote processing device, and
        ii. executing a method for calculation of respiratory training intensity in a dataset representing a subject breathing as disclosed elsewhere herein.

The respiratory sensing system is preferably arranged for retrofitting / installation on an existing respiratory training and/or testing devices, i.e. typical a device which has an inspiratory airway and an expiratory airway and wherein the presently disclosed electronic sensor can be installed such that inspiratory and/or expiratory pressure data can be obtained for analysis according to the approach described herein. For example, the respiratory sensing system may enable respiratory pressure sensing, calculation of respiratory training intensity in a dataset representing a subject's breathing, and the calculation of respiratory muscle power vs. time of at least one breath of the subject, and/or the respiratory muscle work of at least one breath of the subject.

The electronic sensor unit may for example be adapted to be positioned in an inspiratory and/or expiratory airway of a respiratory training and testing device.

However, in specific examples, the respiratory sensing system may comprise a tubular connector arranged for being connected to an inspiratory and/or expiratory airway of a respiratory training and testing device, The tubular connector is preferably arranged to contain the electronic sensor unit, such that said sensor unit measures pressure within the connector portion. The tubular connector is preferably configured to connect to an end of inspiration and/or expiration pathway. The tubular connector may further comprise a mouthpiece.

In yet a further aspect, the present disclosure relates to a respiratory training and testing system for exercising and analysing respiration of a subject comprising:

-   a breathing unit comprising:

    •   a mouthpiece connected to:

        -   at least one inhalation airway having an adjustable inhalation airflow resistance,
        -   at least one exhalation airway having an adjustable exhalation airflow resistance,

- an electronic sensor unit comprising

  - at least one pressure sensor for measuring air pressure in the mouthpiece, and
  - a processing unit for transmitting pressure data,

- a computer program having instructions, which, when executed by a remote processing device, such as a smartphone, cause the processing device to:

  - obtaining said pressure data via a receiver on the remote processing device, preferably continuously obtaining said pressure data, and
  - executing a method for calculation of respiratory training intensity in a dataset representing a subject breathing as disclosed elsewhere herein.

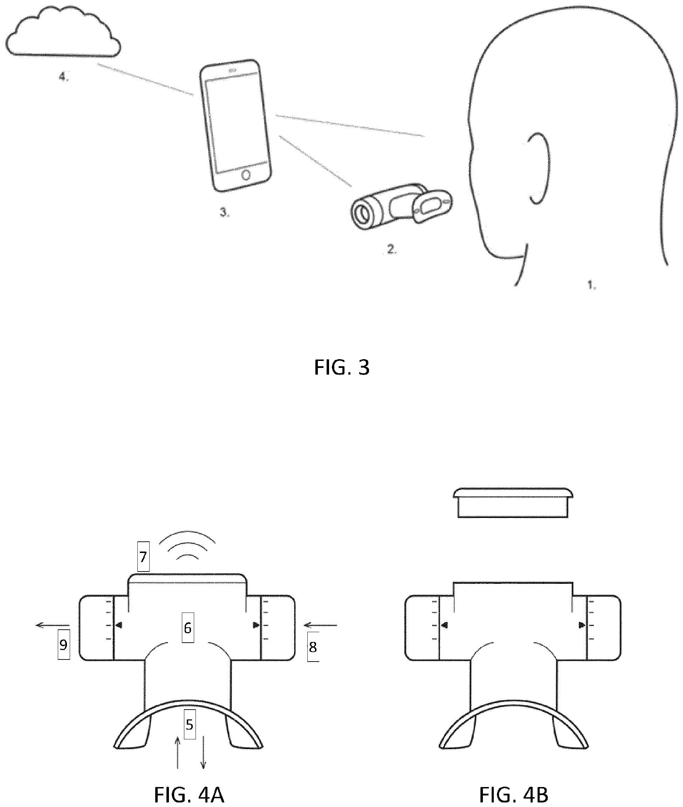

The breathing unit, with the mouthpiece and the electronics sensor unit, is preferably portable as also illustrated in figs. 4-6, thereby in practise functioning as a personal respiratory training and testing device which can be used during active workout.

In one embodiment the electronic sensor unit comprises a storage medium, i.e. in the form of memory (RAM) or other similar digital storage medium, for storing pressure data, at least temporarily, such that pressure data can be stored on the breathing unit if there is no connection to the remote processing device for a period of time, e.g. 30 minutes or 1-5 hours, for example during a training session where for example a smartphone is not necessarily carried by the user. When connection is re-established to the remote processing device, stored pressure data can be transmitted from the breathing unit and to the remote processing device.

In a preferred embodiment of the present disclosure, the computer program is having instructions, which, when executed by the remote processing device, cause the processing device to display instantaneous and/or accumulative calculated power and/or work data on a screen of the remote processing device. By the use of the presently disclosed respiratory training and testing system a subject may obtain accurate information related to respiratory training intensity in a dataset.

An example of a device that is suitable for use along with the presently disclosed methods and/or as part of the presently disclosed respiratory training and testing system is disclosed in "Respiratory device and system for exercising and analysing respiration of a subject" filed 13.07.2017 and pending as WO 2018/011358. WO 2018/011358.

## Description of drawings

**Fig. 1** shows an example of a respiratory pressure-flow-power calculation according to a specific embodiment of the present disclosure.

**Fig. 2** shows an example of a maximum respiratory pressure of a subject with respect to the lung volume of the subject according to a specific embodiment of the present disclosure.

**Fig. 3** shows an overview of a respiratory training and testing system according to a specific embodiment of the present disclosure.

**Figs. 4A-B** show a respiratory training and testing device having two individually adjustable air flow resistances, according to a specific embodiment of the present disclosure.

**Figs. 5A-D** show different external views of an embodiment of a respiratory training and testing device, according to some embodiments of the present disclosure.

**Figs. 6A-C** show different external views of an embodiment of a respiratory training and testing device, according to some embodiments of the present disclosure.

## Definitions

As used herein the term "breath" refers to the part of the breathing cycle that consist or comprises of an inspiration and/or an expiration. A breath as used herein may thereby consist of an inspiration, an expiration, an inspiration followed by an expiration or an expiration followed by an inspiration, potentially combined with a respiratory pause for example between inspiration and expiration.

As used herein, the term "respiratory muscle training" (RMT) refers to a technique that aims to improve the function of the respiratory muscles through specific exercises. RMT is beneficial for a wide variety of subjects, including subjects suffering from respiratory conditions such as asthma, bronchitis, emphysema and COPD. However, RMT may also be part of a subject's sports training, such as in order to increase the endurance and/or strength and/or power of respiratory muscles. Benefits of respiratory muscle training together with methods and systems for the implementation of RMT are described in further detail in McConnell, A. Respiratory Muscle Training: Theory and Practice. Elsevier, 2013.

As used herein, the term remote processing device should be construed broadly not only as a physical device but also including e.g. remote servers, clouds or web-based content management systems. It should be noted that it is the device of the legitimate user i.e. a server of the online service is not included. Preferably, the remote processing device is remote from the electronic sensor unit. However, the subject may be in possession of the remote processing device, alternatively it may be located remote from the subject, such as wherein a trainer or health professional is in possession of the remote processing device. Examples of remote processing devices include the above mentioned examples and also a mobile device, such as a smart phone, a tablet computer, a smart watch, smart glasses, or a wearable device.

As used herein, the term pressure sensor refers to a device for pressure measurement of gases or liquids. The pressure sensor may for example be a gauge pressure sensor, a differential pressure sensor, a vacuum pressure sensor or a sealed pressure sensor, or a combination thereof. It should be noted that as used herein, a pressure sensor may comprise multiple sensing units, and that the sensor or the processing unit is configured to calculate a pressure based on pressure data from multiple sensing units.

**Detailed description**

The present disclosure relates to methods and systems for obtaining accurate respiratory training intensity information, i.e. inspiratory training intensity information and/or expiratory training intensity information.

In a first aspect the present disclosure relates to a computer implemented method for calculation of respiratory training intensity, such as inspiratory training intensity and/or expiratory training intensity. The calculations are typically based on a dataset representing a subject breathing through a respiratory training unit, wherein said device preferably has separate inhalation and exhalation airways having predefined respiratory resistances.

In some embodiments of the present disclosure, the computer implemented method comprises a step of calculating the respiratory muscle power of at least a part of one breath of the subject, such as at least one complete breath of the subject. The respiratory muscle power is typically calculated for a multiple time points, and thereby the respiratory muscle power may be calculated vs. time, such as during at least one breath of the subject, or during a training session. The respiratory muscle power is typically calculated based on instantaneous measurement values, such as an instantaneous flow rate and an instantaneous pressure. In such cases, the respiratory muscle power may represent a momentary value of the power, however the respiratory muscle power may alternatively or additionally be based on average measurement values, such as an average flow rate and/or an average pressure. In such cases the respiratory muscle power may represent an average pressure over a predetermined time interval, and/or number of breaths.

In some embodiments of the present disclosure, the computer implemented method comprises a step of calculating respiratory muscle work of at least a part of one breath of the subject, more preferably at least one breath of the subject. Respiratory muscle work may further be calculated for other lengths, such as for a predetermined amount of time, number of breaths, or for an entire training session, or part thereof.

In a preferred embodiment of the present disclosure, the calculation of a subject's respiratory training intensity (power and/or work) is a real-time process carried out using a stream of data continuously received. The data may be obtained by a pressure sensor, for example located in a mouthpiece of a respiratory training unit, configured for measuring inspiratory and/or expiratory pressures in an airway of said respiratory training unit. The method is thereby applicable to continuous calculation of inspiratory muscle training intensity and/or expiratory muscle training intensity, for example depending on the type of training benefits desired.

The presently disclosed method may in certain examples be implemented by the use of a respiratory training system through which a subject can select whether training of inspiratory muscles, training of expiratory muscles or training of both types of muscles are desired. In response to said selection, the system may obtain the relevant pressure and/or flow rate data, i.e. related to inspiration and/or expiration, and perform calculations based on these values. In general, the obtained data represent a subject's breathing through the inhalation and/or exhalation airways, preferably wherein the airways each has a predefined respiratory resistance.

In an embodiment of the present disclosure, the dataset comprises pressure data vs. time acquired in connection with at least one of the inhalation and exhalation airways. A pressure sensor may be located in a mouthpiece of a respiratory training unit, such that said pressure sensor is connected to at least one of the inhalation and exhalation airways. In further embodiments of the present disclosure, the respiratory training system comprises multiple pressure sensors, configured for measuring different pressure ranges. The respiratory training system is typically configured to derive a flow rate by pressure measurements. However, in certain embodiments of the present disclosure, the respiratory training system comprises a pressure sensor and a flow rate sensor. Thus, in an embodiment of the present disclosure, the dataset comprises flow rate vs. time acquired in connection with at least one of the inhalation and exhalation airways.

In some embodiments of the presently disclosed method, said method is related to calculation of respiratory training intensity. Respiratory training intensity may be quantitatively and/or qualitatively determined in a number of ways, and characterized by a number of parameter values. For example, the respiratory training intensity may be described by respiratory muscle power, such as the average and/or peak respiratory muscle power per breath, or by respiratory muscle work, such as the respiratory muscle work per breath.

*Power*

In further embodiments of the present disclosure, the computer implemented method comprises a step of calculating the average respiratory muscle power of at least one breath, a predefined time interval, a training session and/or a part thereof. The computer implemented method may for example be configured to calculate the average respiratory muscle power during an inspiration, an expiration or a breath (an inspiration and/or an expiration).

In a specific embodiment of the present disclosure, the computer implemented method comprises a step of calculating the peak respiratory muscle power of at least one breath, a predefined time interval, a training session and/or a part thereof. The computer implemented method may for example be configured to calculate the peak respiratory muscle power during an inspiration, an expiration or a breath (an inspiration and/or an expiration).

In some embodiments of the present disclosure, the computer implemented method comprises a step of calculating the average peak respiratory muscle power of a plurality of breaths of the subject. The computer implemented method may for example be configured to calculate, for each breath, the peak respiratory muscle power. The computer implemented method may further be configured to calculate, based on multiple peak respiratory muscle power values, each obtained from a single breath, an average thereof, for example during a training session of for a predetermined amount of time.

In a specific embodiment of the present disclosure, the computer implemented method comprises a step of calculating the respiratory rate of change of pressure vs. time of at least one breath of the subject and/or the maximum rate of change of pressure of at least one breath of the subject. The respiratory rate of change of pressure may be the inspiratory rate of change of pressure and/or the expiratory rate of change of pressure. The respiratory rate of change of pressure may for example provide information related to how quickly the muscles are able to provide a force (pressure) during loaded muscle contractions, i.e. it may be considered as the speed of pressure generation.

*MRP*

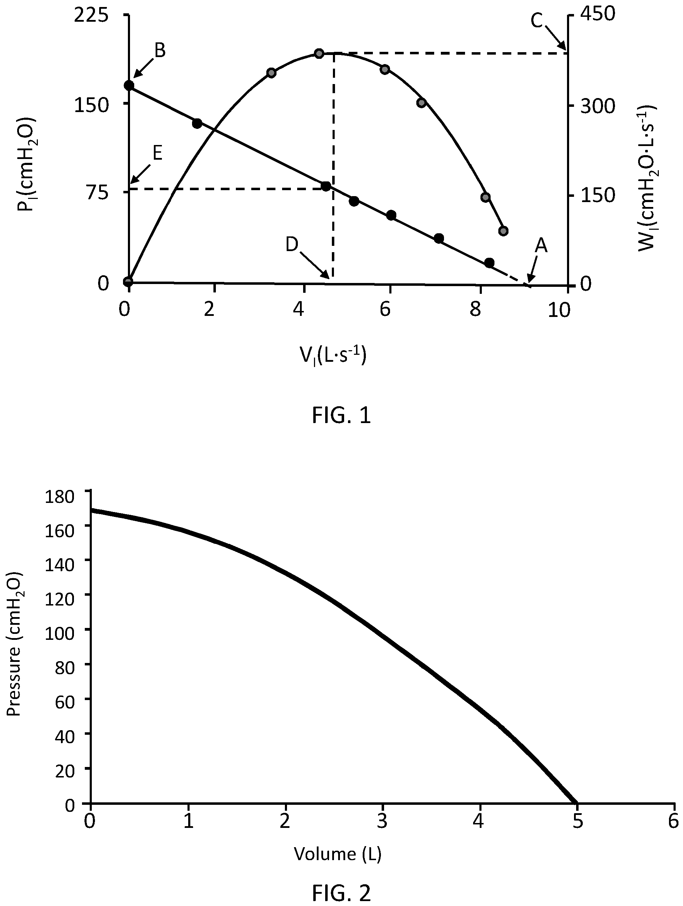

The (maximal) pressure generating capacity of the respiratory muscles is flow dependent. Line A-B in Figure 1 shows an example of a linear regression between a pressure load and the capacity to generate airflow. The pressure and flow generating capacity may for example be measured by requiring a subject to make a series of inspirations at maximal inspiratory effort against a range of fixed pressure loads or flow resistances.

Typically a linearly decreasing flow rate (e.g. measured in $L \cdot s^{-1}$) is seen with increasing pressure loads (e.g. measured in $cmH_2O$). Mathematically and physiologically, the product of pressure and flow is power. Accordingly, the respiratory muscle power of the inspiratory and/or the expiratory muscles can be estimated as described above. The peak respiratory muscle power (C in Figure 1) typically occurs at the mid-point of the pressure-flow relationship. Thus, the optimal flow rate (D in Figure 1) and pressure (E in Figure 1) for the production of maximal power are 50% of the values at A and B, respectively.

While the dependency between the flow generating capacity of the respiratory muscles, such as the inspiratory muscles and/or the expiratory muscles, and maximal pressure generating capacity can be determined as described above, an alternative way of estimating the same is based on the fact that the gradient of the maximal pressure generating capacity of the inspiratory muscles and/or the expiratory muscles vs flow rate is relatively uniform in healthy human beings, typically ~17.5 units. Accordingly, the pressure at any point on a line describing the relationship between the maximal flow generating capacity and the pressure, as shown in Figure 1, line A-B, can be estimated by measuring the flow rate generated by a user during inspiration and/or expiration at a single pressure load. A subject may as such use a respiratory training unit set at any respiratory resistance setting, i.e. any pressure load. For example, the maximum respiratory pressure (MRP), such as the maximum inspiratory pressure or the maximum expiratory pressure, may be estimated by measuring the flow rate and the respiratory pressure at a specific respiratory pressure setting of a respiratory training unit (pressure load), and thereafter deriving MRP by

$$MRP = (\dot{V}_I \cdot R_p) + P_V \qquad (1)$$

where MRP is the maximal (static) respiratory pressure; $\dot{V}_I$ = measured flow rate; $P_V$ = measured pressure at $\dot{V}_I$; $R_P$ is a respiratory pressure constant, typically 17.5.

In an embodiment of the present disclosure, the respiratory pressure constant is between 10 and 25, such as between 13 and 22, more preferably between 16 and 19, most preferably 17.5.

Consequently the maximum inspiratory pressure (MIP) may be estimated by measuring the flow rate and the inspiratory pressure, at maximum inspiratory effort and at a set pressure load, and thereafter calculating MIP through the above mentioned formula. Similarly, the maximum expiratory pressure (MEP) may be estimated by measuring the flow rate and the expiratory pressure, at maximum expiratory effort and at a set pressure load, and thereafter calculating MEP by the above mentioned formula.

Thereby, in some embodiments of the present disclosure, the computer implemented method comprises a step of calculating a maximum respiratory pressure of the subject, by multiplying a respiratory pressure constant to a flow rate obtained at maximum respiration effort of the subject, such as at a set pressure load, and thereafter adding a value of pressure measured at said maximum respiration.

In another embodiment of the present disclosure, the computer implemented method comprises a step of calculating a maximum inspiratory pressure of the subject, by multiplying a respiratory pressure constant to a flow rate obtained at maximum inspiratory effort of the subject, such as at a set pressure load, and thereafter adding a value of pressure measured at said maximum inspiration.

In another embodiment of the present disclosure, the computer implemented method comprises a step of calculating a maximum expiratory pressure of the subject, by multiplying a respiratory pressure constant to a flow rate obtained at maximum expiratory effort of the subject, such as at a set pressure load, and thereafter adding a value of pressure measured at said maximum inspiration.

It should be noted that while a respiratory constant of 17.5 is typically accurate for use to calculate respiratory values of an average healthy subject, other values of the respiratory constant may be more suitable depending on for example the respiratory strength of the subject, age, weight, length, sex, fitness level and/or any existing medical conditions, such as asthma or Chronic obstructive pulmonary disease (COPD). It is however a strong preference that the respiratory constant, $R_P$, is between 10 and 25, more preferably between 12.5 and 22.5, yet more preferably between 15 and 20, even yet more preferably between 16 and 19, once even yet more preferably between 17 and 18, most preferably 17.5.

In a specific embodiment of the present disclosure, the respiratory constant, $R_P$, is estimated based on one or more respiratory properties of the subject, such as any property that may affect the respiratory capabilities of the subject, including the age, weight, length, sex, fitness level and/or existing medical conditions of the subject, or respiratory measurements of the subject, such as respiratory muscle power vs. time of at least one breath and/or respiratory muscle work vs. time of at least one breath. The method of the present disclosure may for example comprise a step of estimating a respiratory constant of the subject based on a regression model or a machine learning model, wherein said models may have been trained based on a number of training datasets, such as wherein each training data set comprises a measured respiratory constant, respiratory properties of an individual and/or respiratory measurements of the individual, such as respiratory muscle power vs. time of at least one breath and/or respiratory muscle work vs. time of at least one breath.

For example, If the pressure and flow measured at a given resistance setting of a respiratory training unit (a set pressure load) are 100 cmH$_2$O and 0.5 l·sec$^{-1}$, respectively, the maximum inspiratory pressure may be given by MIP = (0.5 · 17.5) + 100 = 109 cmH$_2$O where 17.5 is used as the respiratory pressure constant.

*MRF*

Similar principles for determining the maximum respiratory pressure may further be used to determine the maximum respiratory flow rate (MRF), such as the maximum inspiratory flow rate (MIF) and/or the maximum expiratory flow rate (MEF). Direct measurements of the maximum respiratory flow rate (i.e. at zero pressure load) is typically associated with significant accuracy problems, instead determining the maximum respiratory flow rate at higher pressure load provides in general a higher accuracy. The maximum respiratory flow rate may be estimated by determining the flow rate and pressure at maximum respiratory effort, at a specific pressure load (e.g. a respiratory resistance setting of a respiratory training unit), and thereafter deriving the MRF through the formula

$$MRF = \dot{V}_I + ( P_V / 17.5) \qquad (2)$$

wherein $\dot{V}_I$ = measured flow rate; $P_V$ = measured pressure at Vi. Similarly, the maximum inspiratory flow rate (MIF) and/or the maximum expiratory flow rate (MEF) may be estimated by the same formula by measuring the flow rate and pressure, at maximum inspiratory or expiratory effort respectively, at a set pressure load.

In an embodiment of the present disclosure, the computer implemented method comprises a step of calculating a maximum respiratory flow rate of the subject, by dividing a pressure measured at maximum respiratory effort of a subject, at a set pressure load, with a respiration flow rate constant, and thereafter adding a value of flow rate measured at said maximum respiration. Similarly, in further embodiments of the present disclosure, the computer implemented method comprises a step of calculating a maximum inspiratory flow rate of the subject, by dividing a pressure measured at maximum inspiratory effort of the subject, at a set pressure load, with a respiration flow rate constant, and thereafter adding a value of flow rate measured at said maximum inspiratory. Further, in another embodiment of the present disclosure, the computer implemented method comprises a step of calculating a maximum expiratory flow rate of the subject, by dividing a pressure measured at maximum expiratory effort of the subject, at a set pressure load, with a respiration flow rate constant, and thereafter adding a value of flow rate measured at said maximum expiratory effort.

For example, if the pressure and flow measured at a given setting of a respiratory training system (a set pressure load) are 15 $cmH_2O$ and 7.5 $l\cdot sec^{-1}$, respectively, the maximum inspiratory pressure may be given by

PIF = 7.5 + (15 / 17.5) = 8.35 $\cdot sec^{-1}$
where 17.5 is used as the respiratory pressure constant.

*Gradient*

In some embodiments of the present disclosure, the computer implemented method comprises a step of calculating a gradient of a slope describing the relationship between the maximum respiratory flow rate and the pressure of the subject by measuring said maximum respiratory flow rate and the pressure at two different respiratory resistances (pressure loads) and thereafter calculating said gradient.

The gradient (slope) of a line describing the maximal pressure generating capacity of the respiratory muscles (e.g. line A-B in Fig. 1) may be determined by measuring the pressure and flow at two separate pressure loads, e.g. two different respiratory resistance settings of a respiratory training system, and thereafter deriving the gradient through the formula

$$\text{Gradient} = (P_F - P_A) / (\dot{V}_A - \dot{V}_F) \tag{3}$$

wherein $\dot{V}_A$ = measured flow at pressure load F; $\dot{V}_{A\,=}$ measured flow at pressure load A; $P_F$ = measured pressure at pressure load F; $P_{A\,=}$ measured pressure at pressure load A, and F and A are two separate pressure loads, for example obtained at two different respiratory resistance settings of a respiratory training system. The gradient is preferably calculated based on pressure and flow values obtained during maximal inspiratory or expiratory effort of the subject, e.g. the gradient can either describe the relationship between pressure and flow during maximal expiration effort of a subject, or the relationship between pressure and flow during maximal inspiratory of a subject.

For example, a respiratory training system may be set to respiratory resistance setting A corresponding to a first pressure load. At maximum inspiratory (or expiratory) effort of a subject at said pressure load, pressure and flow is measured to 15 $cmH_2O$ and 7.5 $l\cdot sec^{-1}$, respectively. Thereafter the respiratory training system may be set to respiratory resistance setting F corresponding to a second pressure load. At maximum inspiratory (or expiratory) effort of the subject at said pressure load, pressure and flow is measured to 100 $cmH_2O$ and 0.5 $l\cdot sec^{-1}$, respectively. The gradient is consequently given by Gradient = (100 - 15) / (7.5 - 0.5) = 12.14.

In a preferred embodiment of the present disclosure, the computer implemented method comprises calculating respiratory muscle power vs. time of at least one breath of the subject. The respiratory muscle power vs. time may in turn be used to derive one or more training intensity indicators. For example, the peak respiratory muscle power per breath, or per any other interval, which is the highest value of the (instantaneous) respiratory muscle power during that time interval. The (instantaneous) respiratory muscle power is the product of the (instantaneous) flow rate and pressure. Further, the average respiratory muscle power may be calculated per breath, or any other interval. The average respiratory muscle power may for example be calculated as the mean of the (instantaneous) power throughout the breath, or any other interval. In an embodiment of the present disclosure, the computer implemented method is configured to calculate the peak respiratory muscle power (PRP) per breath or any other interval, such as a predefined time interval, a respiratory training session or part thereof, by calculating the highest (instantaneous) respiratory muscle power value, per breath or said other interval. In another embodiment of the present disclosure, the computer implemented method is configured to calculate the average respiratory muscle power, per breath or any other interval, such as a predefined time interval, a respiratory training session or part thereof, by calculating the mean of the (instantaneous) respiratory muscle power, per breath or said other interval.

In a further embodiment of the present disclosure, the computer implemented method is configured to calculate a session average of the peak respiratory muscle power (PRP) per breath and/or the average respiratory muscle work per breath. The length of the session may be a predefined amount of time, a predefined number of exercises, a predefined number of breaths, and/or until fatigue is identified.

In a specific embodiment of the present disclosure, the computer implemented method comprises estimating a peak respiratory muscle power of the subject by multiplying half the maximum respiratory pressure by half the maximum respiratory flow rate. It has been identified that the theoretical peak respiratory muscle power of a subject occurs at around half the maximum respiratory pressure and around half the maximum respiratory flow rate, therefore these values allow for a good approximation of the theoretical peak respiratory muscle power. In a preferred embodiment of the present disclosure, the computer implemented method is configured to calculate the peak respiratory muscle power, such as the maximum inspiratory power and/or the maximum expiratory power, by multiplying the maximum respiratory pressure, such as the maximum inspiratory pressure and/or the maximum expiratory pressure respectively, with the maximum respiratory flow rate, such as the maximum inspiratory flow rate and/or the maximum expiratory flow rate respectively, and a peak respiratory muscle power factor, wherein the peak respiratory muscle power factor is between 0.1 and 0.4, more preferably between 0.15 and 0.35, yet more preferably between 0.2 and 0.3, most preferably 0.25. Thereby, peak respiratory muscle power may be calculated by

$$PRP = MRF \cdot MRP \cdot F_P \tag{4}$$

Wherein PRP is the peak respiratory muscle power; MRF is maximum respiratory flow rate; MRP is the maximum respiratory pressure; $F_P$ is the peak respiratory muscle power factor, typically 0.25.

*Subject-specific power target*

In a specific embodiment of the present disclosure, the computer implemented method comprises a step of calculating a subject specific respiratory muscle power target for at least one breath, a predetermined amount of time, a training session or a part thereof. Said calculations may be based on the respiratory muscle power vs. time relative to a target respiratory muscle power of the subject, such as a fraction of the peak respiratory muscle power. The subject-specific power target may for example be a predetermined fraction of the peak respiratory muscle power, such as 50%, 60%, or 80%. A respiratory training system may for example be configured to display the subject-specific power target to a subject using the system, together with a (continuously updated) peak respiratory muscle power per breath, or an average respiratory muscle power per breath during a training session.

*Subject-specific power factor*

In a specific embodiment of the present disclosure, the computer implemented method comprises a step of calculating a subject specific respiratory muscle power factor based on an average peak respiratory muscle power of a plurality of breaths of the subject relative to a peak respiratory muscle power of the subject. The subject-specific power factor thereby gives an indication of how near the subject was at reaching the subject-specific maximum, i.e. the peak respiratory muscle power.

The subject specific respiratory muscle power factor may be a subject specific inspiratory power factor and/or a subject specific expiratory power factor. For example, the subject specific inspiratory power factor may be calculated based on an average maximum inspiratory power of a plurality of breaths of the subject relative to a maximum inspiratory power of the subject. Additionally or alternatively, the subject specific respiratory muscle power factor may be a subject specific expiratory power factor that is calculated based on an average maximum expiratory power of a plurality of breaths of the subject relative to a maximum expiratory power of the subject.

*Work*

Maximal pressure generating capacity of the respiratory muscles (inspiratory muscles and expiratory muscles) varies across the lung volume (Figure 2). At a given lung volume, respiratory pressure (P) can be calculated using the equation:

$$P = MRP - MRP \cdot (V^2 + 2 \cdot V) / (V_{INMAX}^2 + 2 \cdot V_{INMAX}) \tag{5}$$

MRP = maximal [static] respiratory pressure (or an inferred value from for example maximum respiratory flow rate and/or flow measured during loaded respiratory efforts)
V = prevailing lung volume
$V_{INMAX}$ = maximum inhaled volume (aka vital capacity)

*Estimation of Theoretical Maximum Work Capacity*

Maximum attainable work for a single breath is the integral of maximum pressure with respect to volume (area under the curve depicted in Figure 2) is given by $W_{MAX}$:

$$W_{MAX} = \int^{VC} PdV \tag{6}$$

$$W_{MAX} = MRP \cdot [(\tfrac{2}{3} \cdot V_{INMAX}{}^2 + V_{INMAX})/(V_{INMAX} + 2)] \tag{7}$$

If, MRP = 160 cmH$_2$O and $V_{INMAX}$ = 4.6L, then maximum attainable work over a breath is: $W_{MAX}$ = 453 cmH$_2$O·L

Over, say, a 30 breath training session, total maximum theoretically predicted work:

$$W_{MAXTOT} = 30 \cdot 453 = 13{,}590 \text{ cmH}_2\text{O·L}$$

$$W_{MAXTOT} = 0.090665 \text{ cmH}_2\text{O·L/Joules} \cdot 13{,}590 \text{ cmH}_2\text{O} \cdot \text{L} = 1{,}232 \text{ Joules}$$

*Note*: 0.090665 is a constant that converts cmH$_2$O·L to Joules

In typical situations the maximum attainable respiratory muscle work is lower than the (theoretical) $W_{MAX}$. One reason for this is that the equation for calculating $W_{MAX}$, as given above, is based upon static inspiratory muscle strength. Pressure generating capacity (dynamic strength) is typically considerably lower. Another reason is that it is typically not possible to work at 100% of $W_{MAX}$ for an entire training session due to the effects of fatigue. Accordingly, for the setting of real-time training intensity targets, as well as for retrospective rating training session quality, a factor of the theoretical $W_{MAX}$, may be used to approximate the physiological maximum work capacity ($W_{MAXphys}$). In other words, the work capacity of a dynamically breathing human being is assumed to be a fraction of their theoretical maximum work capacity. It has been shown that 0.5 $W_{MAX}$ is a good approximation of $W_{MAXphys}$ for a number of breaths completed in a given session, such as 30. Said fraction may be between 0.1 and 0.9, more preferably between 0.2 and 0.8, yet more preferably between 0.3 and 0.7, even yet more preferably between 0.4 and 0.6, most preferably 0.5.

The physiological maximum work capacity for a breath may be calculated by

$$W_{MAXphys} = 0.5 \cdot MRP \cdot [(\tfrac{2}{3} \cdot V_{INMAX}{}^2 + V_{INMAX})/(V_{INMAX} + 2)] \tag{8}$$

while the physiological maximum work capacity for a training session is given by

$$W_{MAXphysTOT} = 0.5 \cdot n \cdot MRP \cdot [(\tfrac{2}{3} \cdot V_{INMAX}{}^2 + V_{INMAX})/(V_{INMAX} + 2)] \tag{9}$$

where n is the number of breaths in the training session. As also described elsewhere herein, the physiological maximum work capacity is typically calculated for a breath, i.e. an inspiration, an expiration or an inspiration and an expiration, or multiple breaths, i.e. multiple inspirations, multiple expiration, or multiple inspirations and expirations.

*ARW*

In a specific embodiment of the present disclosure, the computer implemented method comprises a step of calculating the accumulated respiratory muscle work for at least one breath of the subject. In a specific embodiment of the present disclosure, the computer implemented method comprises a step of calculating the accumulated respiratory muscle work vs. time for at least one breath of the subject. It should be noted that the accumulated respiratory muscle work may be either accumulated inspiratory work and/or accumulated expiratory work. The accumulated respiratory muscle work may comprise a factor between 0 and 1 to calculate the physiological maximum work capacity, typically 0.5.

Respiratory muscle work per breath ($W_B$) may be calculated by integrating measured respiratory muscle power (pressure · flow) with respect to time, i.e. by summing the product of pressure and flow for each sample and then multiplying the sum by the sampling interval. Alternatively, $W_B$ can be calculated as the integral of pressure with respect to volume (area under the curve in Figure 2). Respiratory muscle work per breath may be the inspiratory work per breath and/or the expiratory work per breath, and the method may further comprise a step of calculating the accumulated work for a training session, based on the work per breath by summing the work for each breath of the training session.

In a specific embodiment of the present disclosure, the computer implemented method comprises a step of calculating the average respiratory muscle work for a plurality of breaths of the subject. It should be noted that the average respiratory muscle work may either be the average inspiratory work and/or the average expiratory work. As described elsewhere herein the average respiratory muscle work per breath may be derived by summing the product of pressure and flow for each sample and then multiplying the sum by the sampling interval. Alternatively, $W_B$ may be calculated as the integral of pressure with respect to volume.

In a specific embodiment of the present disclosure, the computer implemented method comprises a step of calculating a subject specific work target of at least one breath based on a maximum respiratory muscle work capacity for one breath of the subject. The maximum respiratory muscle work capacity may for example be based on a physiological respiratory muscle work capacity, such as the maximum respiratory muscle work capacity multiplied with a factor, typically 0.5. The subject specific work target is preferably a subject specific work factor of the physiological respiratory muscle work capacity. The work factor may for example be 50%, 70%, or 90%, such that the subject specific work target reflects a training intensity target of the subject. The subject specific work target ($T_W$) may be calculated by the following formula

$$T_W = F \times 0.5 \times MRP \times [(\tfrac{2}{3}.V_{INMAX}{}^2 + V_{INMAX})/(V_{INMAX} + 2)] \tag{10}$$

wherein F is the subject specific work factor, and 0.5 has been used as a factor to calculate the physiological respiratory muscle work capacity. Depending on the type of training (inspiratory training, expiratory training, or inspiratory and expiratory training), the subject specific work target may be based on inspiratory data, expiratory data, or both.

*Subject specific work feedback*

In a specific embodiment of the present disclosure, the computer implemented method comprises a step of calculating a subject specific work feedback of at least one breath based on the accumulated respiratory muscle work vs. time relative to a maximum respiratory muscle work capacity for at least one breath of the subject. The maximum respiratory muscle work capacity may for example be the physiological respiratory muscle work capacity, i.e. the maximum respiratory muscle work capacity multiplied with a compensation factor, typically 0.5. The subject specific work feedback ($F_W$) may be calculated by

$$F_W = 100 \times W_A/W_{MAXphysTOT} \tag{11}$$

wherein $W_A$ is the accumulated respiratory muscle work, typically of a training session, and $W_{MAXphys}$ the physiological respiratory muscle work capacity of said training session, i.e. the physiological respiratory muscle work capacity time the number of breaths of the training session.

In a specific embodiment of the present disclosure, the step of calculating a subject specific work feedback is based on an accumulated respiratory muscle work of a plurality of breaths of the subject relative to a maximum respiratory muscle work capacity of said plurality of breaths. The subject specific work feedback may be calculated by the formula $F_W = 100 \times W_{TOT}/W_{MAXphysTOT}$, and may be based on inspiratory work, expiratory work, or a combination thereof, for example depending on the desired training type.

The method may further be configured to classify the subject-specific work feedback into an intensity rating, such as LOW, MEDIUM and HIGH. For example wherein $F_W < 35$ results in the rating = "LOW", $F_W$ between 35 and 65 results in the rating = "MEDIUM", $F_W > 65$ results in the rating = "HIGH".

*Respiratory sensing system*

In a further aspect, the present disclosure relates to a respiratory sensing system. The respiratory sensing system may be configured for retrofitting of respiratory training and testing devices. The respiratory sensing system may comprise an electronic sensor unit and/or a software application aka a computer program (product). In an embodiment of the present disclosure, the electronic sensor comprises at least one pressure sensor and a processing unit. The respiratory sensing system may comprise a single pressure sensor or multiple having different measurement ranges and/or resolutions. The at least pressure sensor is preferably adapted for measuring air pressure in an inspiratory and/or expiratory airway of the respiratory training and testing device. Therefore the size of the pressure sensor is preferably sufficiently small such that the pressure sensor may be accommodated by an inspiratory and/or expiratory airway of a respiratory training and testing device, without obstructing air flow. The electronic sensor unit may further comprise a processing unit for transmitting pressure data, such as wirelessly transmitting pressure data.

In a preferred embodiment of the present disclosure, the electronic sensor unit is detachably mounted to a housing of the breathing unit. Preferably wherein the attachment connection between the breathing unit and the electronic sensor unit is airtight. The detachable electronic sensor unit may for example comprise means for snap-fitting to a main unit of the breathing unit. Thereby the detachable electronic sensor unit may comprise one part of a snap connection, such as a male part, and the main unit may comprise another part of the snap connection, such as a female part.

In a preferred embodiment of the present disclosure, the respiratory sensing system comprises a software application executable on a remote processing device, such as a smartphone. I.e. similar to a computer program (product) having instructions, executable by a remote processing device, such as a smartphone. The software application / computer program is preferably configured for continuously obtaining pressure data via a receiver on the remote processing device, and/or for executing a method for calculation of respiratory training intensity in a dataset representing a subject breathing as disclosed elsewhere herein.

The respiratory sensing system is preferably arranged for retrofitting of respiratory training and devices. For example, the respiratory sensing system may enable respiratory pressure sensing, calculation of respiratory training intensity in a dataset representing a subject's breathing, and the calculation of respiratory muscle power vs. time of at least one breath of the subject, and/or the respiratory muscle work of at least one breath of the subject.

The electronic sensor unit may for example be adapted to be positioned in an inspiratory and/or expiratory airway of a respiratory training and testing device.

However, in specific examples, the respiratory sensing system may comprise a tubular connector arranged for being connected to an inspiratory and/or expiratory airway of a respiratory training and testing device, The tubular connector is preferably arranged to contain the electronic sensor unit, such that said sensor unit measures pressure within the connector portion. The tubular connector is preferably configured to connect to an end of inspiration and/or expiration pathway. The tubular connector may further comprise a mouthpiece.

*Respiratory training and testing system*

In yet a further aspect, the present disclosure relates to a respiratory training and testing system for exercising and analysing the respiratory muscles of a subject. The respiratory muscle training and testing system typically comprises a breathing unit, through which a subject can breathe, such as perform an inspiration, an expiration, or both. The breathing unit may comprise a mouthpiece, an electronic sensor and/or a software application. It is a preference that the mouthpiece is connected to at least one airway, more preferably at least one inspiration (inhalation) airway and an expiration (exhalation). It is a further preference that the airway(s) has adjustable airflow resistance, such as at least one inhalation airway having an adjustable inhalation airflow resistance, and at least one exhalation airway having an adjustable exhalation airflow resistance, i.e. such that the inhalation airflow resistance can be adjusted independently of the exhalation airway resistance - and vice versa. The electronic sensor unit may comprise at least one pressure sensor for measuring air pressure, the sensor unit may for example be configured to measure air pressure in a mouthpiece, additionally or alternatively, the electronic sensor may be configured to measured air pressure in the airway(s), such as an inhalation airway and/or an exhalation airway. Further, the

Fig. 3 shows the general concept of the presently disclosed respiratory training and testing system for exercising and analysing respiration of a subject. As shown, the system may comprise a breathing unit (2), preferably with individually adjustable air flow resistances on inhaled and exhaled air. The breathing unit holds an electronic sensor unit, preferably in the mouthpiece, which measures exercise session data, such as air pressure, and/or time, and/or duration and/or air flow resistance settings. The electronic sensor unit may in one embodiment store the measured data locally. The unit (2) may also perform some local calculations including a dataset representing the breathing of the subject, such as the breathing through separate inhalation and exhalation airways having predefined respiratory resistances. The data and/or calculations may include flow rate, pressure, power, work, respiratory volume excursions and respiratory muscle strength. The same calculation may alternatively, or additionally, be performed on a remote processing device (3), such as a mobile phone. The system is preferably configured to provide respiratory exercise feedback to the subject (1), such as by displaying text or graphics on a display of the remote processing device (3), by audio by a speaker of the remote processing device and/or a tactile indicator or the remote processing device. Exercise data storage, data analysing, session recommendations, and setup can be located both on the system itself, such as the breathing unit or the remote processing device, and/or on a connected platform (4).

Figs. 4A-B disclose embodiments of an example of a breathing unit comprising a mouthpiece 5 that creates an airtight connection to the mouth and enables breathing through the device. The breathing unit in fig. 4 is clearly portable as seen from the size comparison in fig. 3 between smartphone 3, breathing unit 2 and head of user 1. The breathing unit comprises a valve housing 6 which comprises two one-way valves 8, 9 that control the air flow so that the inhaled air passes through one adjustable resistance 8 and the exhaled air passes through another adjustable resistance 9, as also indicated by arrows in fig. 4A. The two adjustable resistance interfaces 8, 9 may comprise predefined settings corresponding to an exact air passage clearance. The user may adjust these settings and read the setting value on the device. An electronic unit 7 is mounted on the device, which may comprise a pressure meter connected to the inside of the valve housing 6. Fig. 4B shows the situation where the electronic unit 7 is dismounted from the valve housing 6. Thereby the valve housing can be cleaned thoroughly. It also makes it possible to use another valve housing with the same electronic unit 7.

In a preferred embodiment of the present disclosure, the respiratory training and testing system comprises a processing unit, that is configured for transmitting pressure data. The pressure data may have been obtained by at least one electronic sensor, for example located within a mouthpiece and/or at least one airway, as disclosed elsewhere herein. The processing unit may for example be configured to communicate over a wireless communication standard, such as Bluetooth®, Wi-Fi™, or mobile broadband. In specific embodiments of the present disclosure, the respiratory training and testing system comprises a software application executable on a remote processing device, such as a smartphone. The software application may be configured for executing the steps of: continuously obtaining pressure data via a receiver on the remote processing device; and executing a computer implemented method for calculation of respiratory training intensity in a dataset, such as a dataset representing a subject breathing through separate inhalation and exhalation airways having predefined respiratory resistances.

In a specific embodiment of the present disclosure, the software application is configured for displaying instantaneous and/or accumulative calculated power and/or work data on a screen of the remote processing device. The software application may, additionally or alternatively be configured to display any type of indicator of respiratory training intensity, subject-specific respiratory capabilities, subject-specific target levels, subject-specific feedback, based on respiratory pressure and/or respiratory muscle work and/or power, as disclosed elsewhere herein. For example the software application may be configured for displaying a subject specific work feedback, subject specific respiratory muscle power target, average respiratory muscle work, accumulated respiratory muscle work, average peak respiratory muscle power, calculated (measured or theoretical) peak respiratory muscle power, calculated (measured or theoretical) maximum respiratory flow rate, calculated (measured or theoretical) maximum respiratory pressure and/or respiratory rate of change of pressure, wherein said parameters may be either the real-time values, averaged values, or accumulated values, and may further be based on measurements of or values calculated to reflect one or more inspirations, one or more expirations, and/or one or more inspirations and expirations.

In a further embodiment of the present disclosure, the remote processing device comprises a processing unit and/or an antenna for communicating with a remote server, such as by telecommunication. The software application is preferably configured for executing transmission of respiratory training data to a remote server. The respiratory training information preferably comprises calculated parameters of a completed respiratory training session, for example related to respiratory muscle power and/or respiratory muscle work. The respiratory training information preferably comprises average respiratory muscle power, peak respiratory muscle power, maximum rate of change of pressure, average peak respiratory muscle power, subject specific respiratory muscle power target, maximum respiratory pressure, maximum respiratory flow rate, subject specific power factor, accumulated respiratory muscle work, average respiratory muscle work, subject specific work feedback and/or subject specific work target.

The remote server is preferably configured to arrange respiratory training information received from multiple subjects into a leader board. The remote server may further be configured to calculate a score for each received respiratory training information based on a combination of the parameters of the respiratory training information.

In a preferred embodiment of the present disclosure, the software application is configured to retrieve the leader board, and the associated data, such as the score provided to each respiratory training information by the remote server and/or one or more parameters of the respiratory training information.

In a further embodiment of the present disclosure, the software application is configured to suggest a specific training routine based on respiratory properties of the subject, such as any property that may affect the respiratory capabilities of the subject, including the age, weight, length, sex, fitness level and/or existing medical conditions of the subject, such as asthma or chronic obstructive pulmonary disease, or respiratory measurements of the subject, such as respiratory muscle power vs. time of at least one breath and/or respiratory muscle work vs. time of at least one breath. The method of the present disclosure may for example comprise a step of predicting the training routine that provides the highest training stimulus based on a regression model or a machine learning model, wherein said models may have been trained based on a number of training datasets, such as wherein each training data set comprises a measured respiratory constant, respiratory properties of an individual and/or respiratory measurements of the individual

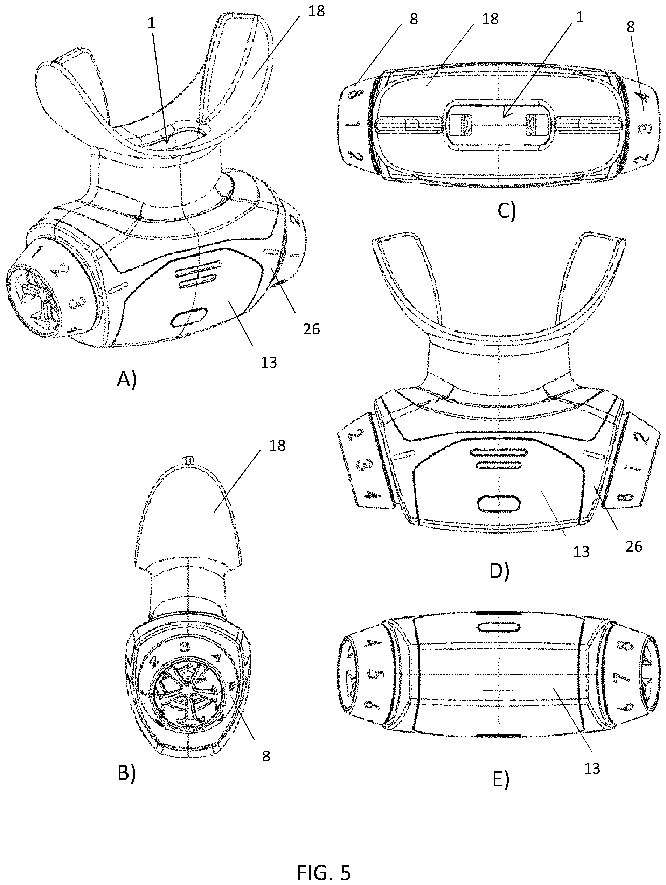

Another embodiment of the breathing device with two individually / independently controllable resistance units is shown in fig. 5. Fig. 5 only shows outside illustrations from different view angles, where fig. 5A is a perspective view, fig. 5B is a side view looking directly at one of the resistance units 8, fig. 5C is a view into the mouthpiece 18 and the mouthpiece airway 1, fig. 5D is a top view where the detachable electronic unit 13 can be seen and fig. 5D is a front view looking directly at the detachable electronic unit 13. The detachable electronic unit 13 is attached to the device housing 26 by means of a snap fit arrangement, such that the electronic unit 13 is solidly attached to the housing 26 during use, but can be detached for cleaning purposes.

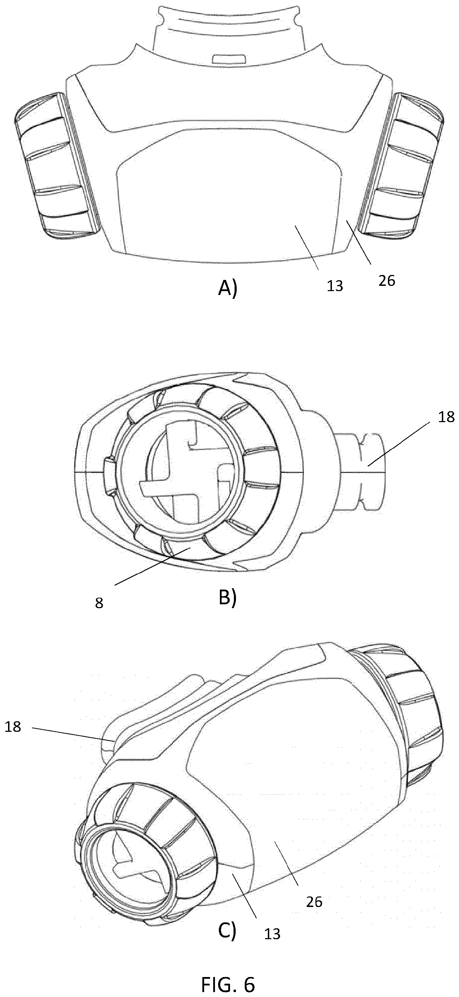

A breathing device according to another embodiment of the present disclosure, is shown in Fig. 6. Fig. 6A is a top view where the detachable electronic unit 13 can be seen. Fig. 6B is a side view looking directly at one of the resistance units 8, and fig. 6A is a perspective view. The breathing devices in figs. 5 and 6 are also clearly portable because the mouthpiece 18 fits in the mouth of a human.

**Claims**

1. A computer implemented method for calculation of respiratory training intensity in a dataset comprising pressure data acquired in connection with at least one of an inhalation and an exhalation airway of a respiratory training or testing device, the dataset representing a subject breathing through said airway having a predefined respiratory resistance, the method comprising the steps of:

   - calculating the respiratory muscle power vs. time of at least one breath of the subject,
   and/or
   - calculating the respiratory muscle work of at least one breath of the subject,

   the method further comprising the steps of:

   - calculating the peak respiratory muscle power of at least one breath of the subject,
   and/or
   - calculating the average peak respiratory muscle power of a plurality of breaths of the subject,

   wherein the calculation of respiratory training intensity is a real-time process carried out using a stream of data continuously received.

2. The method of claim 1, comprising the step of calculating the respiratory rate of change of pressure vs. time of at least one breath of the subject and/or the maximum rate of change of pressure of at least one breath of the subject.

3. The method of any of the preceding claims, comprising the step of calculating a subject specific respiratory muscle power target for at least one breath based on the respiratory muscle power vs. time relative to a target respiratory muscle power of the subject, such as a fraction of the peak respiratory muscle power.

4. The method of any of the preceding claims, comprising the step of calculating a maximum respiratory pressure of the subject, by multiplying a respiratory pressure constant to a flow rate obtained at maximum respiration of the subject, and thereafter adding a value of pressure measured at said maximum respiration.

5. The method of any of the preceding claims, comprising the step of calculating a maximum respiratory flow rate of the subject, by dividing a pressure measured at maximum respiration of the subject with a respiration flow rate constant, and thereafter adding a value of flow rate measured at said maximum respiration.

6. The method of any of the preceding claims, comprising calculating a gradient of a slope describing the relationship between the maximum respiratory flow rate and the pressure of the subject by measuring said maximum respiratory flow rate and the pressure at two different respiratory resistances and thereafter calculating said gradient.

7. The method of any of the preceding claims, comprising estimating a peak respiratory muscle power of the subject by multiplying half the peak respiratory muscle power by half the maximum respiratory flow rate.

8. The method of any of the preceding claims, comprising the step of calculating a subject specific power factor based on average peak respiratory muscle power of a plurality of breaths of the subject relative to a peak respiratory muscle power of the subject
   and/or

   comprising the step of calculating the accumulated respiratory muscle work vs. time for at least one breath of the subject,
   and/or
   comprising the step of calculating the average respiratory muscle work for a plurality of breaths of the subject.

9. The method of any of the preceding claims, comprising the step of calculating a subject specific work feedback of at least one breath based on the accumulated respiratory muscle work vs. time relative to a maximum respiratory muscle work capacity for one breath of the subject; and/or comprising the step of calculating a subject specific work feedback based an accumulated respiratory muscle work of a plurality of breaths of the subject relative to a maximum respiratory muscle work capacity of said plurality of breaths.

**10.** A respiratory sensing system for a respiratory training or testing device having an inspiratory and/or and expiratory airway, the sensing system comprising:

  - an electronic sensor unit comprising;

    - at least one pressure sensor for measuring air pressure in the inspiratory airway and/or the expiratory airway of the respiratory training and testing device, and
    - a processing unit for transmitting pressure data

    - a computer program having instructions, which, when executed by a remote processing device, such as a smartphone (3), cause the processing device to:

    i. continuously obtaining said pressure data via a receiver on the remote processing device, and
    ii. executing the method of any of the preceding claims.

**11.** The respiratory sensing system of claim 10, wherein the computer program is having instructions, which, when executed by the remote processing device, cause the processing device to display instantaneous and/or accumulative calculated power and/or work data on a screen of the remote processing device.

**12.** A respiratory training and testing system for exercising and analysing respiration of a subject (1) comprising:

  - a breathing unit (2) comprising:

    • a mouthpiece (18) connected to:

      - at least one inhalation airway having an adjustable inhalation airflow resistance (8),
      - at least one exhalation airway having an adjustable exhalation airflow resistance (9),

    • an electronic sensor unit (7, 13) comprising

      - at least one pressure sensor for measuring air pressure in the mouthpiece, and
      - a processing unit for transmitting pressure data

  - a computer program having instructions, which, when executed by a remote processing device, such as a smartphone (3), cause the processing device to:

    - continuously obtaining said pressure data via a receiver on the remote processing device, and
    - executing the method of any of the preceding claims 1-9.

**13.** The respiratory training and testing system of claim 12, wherein the computer program is having instructions, which, when executed by the remote processing device, cause the processing device to display instantaneous and/or accumulative calculated power and/or work data on a screen of the remote processing device.

**14.** A system for calculation of respiratory training intensity in a dataset representing a subject breathing, comprising a non-transitive, computer-readable storage device for storing instructions that, when executed by a processor, performs a method for calculation of respiratory training intensity in a dataset representing a subject's breathing according to any one of the claims 1-9.

**15.** A computer program having instructions which when executed by a computing device or system cause the computing device or system to calculate respiratory training intensity in a dataset representing a subject's breathing according to any one of the claims 1-9.

**Patentansprüche**

**1.** Computerimplementiertes Verfahren zur Berechnung der Atemtrainingsintensität in einem Datensatz, der Druckdaten umfasst, die in Verbindung mit mindestens einem von einem Inhalations- und einem Exhalationsatemweg einer Atemtrainings- oder -testvorrichtung erfasst werden, wobei der Datensatz ein Subjekt repräsentiert, das durch den einen vordefinierten Atemwiderstand aufweisenden Atemweg atmet, wobei das Verfahren die folgenden Schritte umfasst:

- Berechnen der Atemmuskelkraft als Funktion der Zeit von mindestens einem Atemzug des Subjekts, und/oder
- Berechnen der Atemmuskelarbeit von mindestens einem Atemzug des Subjekts,
wobei das Verfahren ferner die folgenden Schritte umfasst:

- Berechnen der Spitzenatemmuskelkraft von mindestens einem Atemzug des Subjekts, und/oder
- Berechnen der durchschnittlichen Spitzenatemmuskelkraft einer Vielzahl von Atemzügen des Subjekts,

wobei die Berechnung der Atemtrainingsintensität ein Echtzeitprozess ist, der unter Verwendung eines kontinuierlich empfangenen Datenstroms durchgeführt wird.

2. Verfahren nach Anspruch 1, umfassend den Schritt des Berechnens der Geschwindigkeit der respiratorischen Druckänderung als Funktion der Zeit von mindestens einem Atemzug des Subjekts und/oder der maximalen Geschwindigkeit der Druckänderung von mindestens einem Atemzug des Subjekts.

3. Verfahren nach einem der vorhergehenden Ansprüche, umfassend den Schritt des Berechnens eines subjektspezifischen Atemmuskelkraftziels für mindestens einen Atemzug basierend auf der Atemmuskelkraft als Funktion der Zeit relativ zu einer Zielatemmuskelkraft des Subjekts, wie einem Bruchteil der Spitzenatemmuskelkraft.

4. Verfahren nach einem der vorhergehenden Ansprüche, umfassend den Schritt des Berechnens eines maximalen respiratorischen Drucks des Subjekts durch Multiplizieren einer Konstante des respiratorischen Drucks mit einer Strömungsgeschwindigkeit, die bei maximaler Atmung des Subjekts erhalten wird, und anschließendes Addieren eines bei der maximalen Atmung gemessenen Wertes eines Drucks.

5. Verfahren nach einem der vorhergehenden Ansprüche, umfassend den Schritt des Berechnens einer maximalen respiratorischen Strömungsgeschwindigkeit des Subjekts durch Dividieren eines bei einer maximalen Atmung des Subjekts gemessenen Drucks mit einer Konstante der Atmungsströmungsgeschwindigkeit und anschließendes Addieren eines bei der maximalen Atmung gemessenen Wertes der Strömungsgeschwindigkeit.

6. Verfahren nach einem der vorhergehenden Ansprüche, umfassend Berechnen eines Gradienten einer Steigung, die die Beziehung zwischen der maximalen respiratorischen Strömungsgeschwindigkeit und dem Druck des Subjekts beschreibt, durch Messen der maximalen respiratorischen Strömungsgeschwindigkeit und des Drucks bei zwei verschiedenen Atemwiderständen und anschließendes Berechnen des Gradienten.

7. Verfahren nach einem der vorhergehenden Ansprüche, umfassend Schätzen einer Spitzenatemmuskelkraft des Subjekts durch Multiplizieren der Hälfte der Spitzenatemmuskelkraft mit der Hälfte der maximalen respiratorischen Strömungsgeschwindigkeit.

8. Verfahren nach einem der vorhergehenden Ansprüche, umfassend den Schritt des Berechnens eines subjektspezifischen Kraftfaktors basierend auf der durchschnittlichen Spitzenatemmuskelkraft von einer Vielzahl von Atemzügen des Subjekts relativ zu einer Spitzenatemmuskelkraft des Subjekts und/oder

umfassend den Schritt des Berechnens der akkumulierten Atemmuskelarbeit als Funktion der Zeit für mindestens einen Atemzug des Subjekts,
und/oder
umfassend den Schritt des Berechnens der durchschnittlichen Atemmuskelarbeit für eine Vielzahl von Atemzügen des Subjekts.

9. Verfahren nach einem der vorhergehenden Ansprüche, umfassend den Schritt des Berechnens einer subjektspezifischen Arbeitsrückmeldung von mindestens einem Atemzug basierend auf der akkumulierten Atemmuskelarbeit als Funktion der Zeit relativ zu einer maximalen Atemmuskelarbeitskapazität für einen Atemzug des Subjekts; und/oder umfassend den Schritt des Berechnens einer subjektspezifischen Arbeitsrückmeldung basierend auf einer akkumulierten Atemmuskelarbeit von einer Vielzahl von Atemzügen des Subjekts relativ zu einer maximalen Atemmuskelarbeitskapazität der Vielzahl von Atemzügen.

**10.** Atemsensorsystem für eine Atemtrainings- oder -testvorrichtung, die einem inspiratorischen und/oder exspiratorischen Atemweg aufweist, wobei das Sensorsystem Folgendes umfasst:

- eine elektronische Sensoreinheit, umfassend;
- mindestens einen Drucksensor zum Messen des Luftdrucks in dem inspiratorischen Atemweg und/oder dem exspiratorischen Atemweg der Atemtrainings- oder -testvorrichtung, und
- eine Verarbeitungseinheit zum Übertragen von Druckdaten
- ein Computerprogramm, das Anweisungen aufweist, die beim Ausführen durch eine entfernte Verarbeitungsvorrichtung, wie ein Smartphone (3), die Verarbeitungseinrichtung zu Folgendem veranlassen:

i. fortlaufendes Erhalten der Druckdaten über einen Empfänger an der entfernten Verarbeitungseinrichtung und
ii. Ausführen des Verfahrens nach einem der vorhergehenden Ansprüche.

**11.** Atemsensorsystem nach Anspruch 10, wobei das Computerprogramm Anweisungen aufweist, die beim Ausführen durch die entfernte Verarbeitungsvorrichtung die Verarbeitungsvorrichtung zum Anzeigen von Augenblicks- und/oder akkumulierter berechneter Kraft und/oder Arbeitsdaten auf einem Bildschirm der entfernten Verarbeitungsvorrichtung veranlassen.

**12.** Atemtrainings- oder -testsystem zum Üben und Analysieren der Atmung eines Subjekts (1), umfassend:

- eine Atmungseinheit (2), umfassend:

• ein Mundstück (18), das verbunden ist mit:

- mindestens einen Inhalationsatemweg, der einen einstellbaren Inhalationsluftstromwiderstand (8) aufweist,
- mindestens einen Exhalationsatemweg, der einen einstellbaren Exhalationsluftstromwiderstand (9) aufweist,

• eine elektronische Sensoreinheit (7, 13), umfassend

- mindestens einen Drucksensor zum Messen des Luftdrucks in dem Mundstück und
- eine Verarbeitungseinheit zum Übertragen von Druckdaten
- ein Computerprogramm, das Anweisungen aufweist, die beim Ausführen durch eine entfernte Verarbeitungsvorrichtung, wie ein Smartphone (3), die Verarbeitungseinrichtung zu Folgendem veranlassen:

- fortlaufendes Erhalten der Druckdaten über einen Empfänger an der entfernten Verarbeitungseinrichtung und
- Ausführen des Verfahrens nach einem der vorhergehenden Ansprüche 1-9.

**13.** Atemtrainings- oder -testsystem nach Anspruch 12, wobei das Computerprogramm Anweisungen aufweist, die beim Ausführen durch die entfernte Verarbeitungsvorrichtung die Verarbeitungsvorrichtung zum Anzeigen von Augenblicks- und/oder akkumulierter berechneter Kraft und/oder Arbeitsdaten auf einem Bildschirm der entfernten Verarbeitungsvorrichtung veranlassen.

**14.** System zum Berechnen der Atemtrainingsintensität in einem Datensatz, der eine Atmung eines Subjekts repräsentiert, umfassend eine nicht transitive, computerlesbare Speichervorrichtung zum Speichern von Anweisungen, die beim Ausführen durch einen Prozessor ein Verfahren zur Berechnung einer Atemtrainingsintensität in einem Datensatz, der die Atmung eines Subjekts repräsentiert, nach einem der Ansprüche 1-9 durchführen.

**15.** Computerprogramm, das Anweisungen aufweist, die beim Ausführen durch eine Rechenvorrichtung oder ein Rechensystem die Rechenvorrichtung oder das Rechensystem zum Berechnen der Atemtrainingsintensität in einem Datensatz, der die Atmung eines Subjekts repräsentiert, nach einem der Ansprüche 1-9 veranlassen.

**Revendications**

1. Procédé mis en œuvre par ordinateur pour calculer l'intensité d'un entraînement respiratoire dans un ensemble de données comprenant des données de pression acquises en relation avec au moins l'une d'une voie aérienne d'inspiration et d'une voie aérienne d'expiration d'un dispositif d'entraînement ou de test respiratoire, l'ensemble de données représentant un sujet respirant à travers ladite voie aérienne ayant une résistance respiratoire prédéfinie, le procédé comprenant les étapes de :

   - calcul de la puissance musculaire respiratoire en fonction du temps d'au moins une respiration du sujet, et/ou
   - calcul du travail musculaire respiratoire d'au moins une respiration du sujet,
   le procédé comprenant également les étapes de :

      - calcul de la puissance musculaire respiratoire maximale d'au moins une respiration du sujet, et/ou
      - calcul de la puissance musculaire respiratoire maximale moyenne d'une pluralité de respirations du sujet,

   dans lequel le calcul de l'intensité d'un entraînement respiratoire est un processus en temps réel effectué en utilisant un flux de données reçues en continu.

2. Procédé selon la revendication 1, comprenant l'étape de calcul du taux de variation respiratoire de pression en fonction du temps d'au moins une respiration du sujet et/ou du taux maximal de variation de pression d'au moins une respiration du sujet.

3. Procédé selon l'une quelconque des revendications précédentes, comprenant l'étape de calcul d'une cible de puissance musculaire respiratoire spécifique au sujet pour au moins une respiration sur la base de la puissance musculaire respiratoire en fonction du temps par rapport à une puissance musculaire respiratoire cible du sujet, telle qu'une fraction de la puissance musculaire respiratoire maximale.

4. Procédé selon l'une quelconque des revendications précédentes, comprenant l'étape de calcul d'une pression respiratoire maximale du sujet, en multipliant une constante de pression respiratoire par un débit obtenu à la respiration maximale du sujet, puis en ajoutant une valeur de pression mesurée à ladite respiration maximale.

5. Procédé selon l'une quelconque des revendications précédentes, comprenant l'étape de calcul d'un débit respiratoire maximal du sujet, en divisant une pression mesurée à la respiration maximale du sujet par une constante de débit respiratoire, puis en ajoutant une valeur de débit mesurée à ladite respiration maximale.

6. Procédé selon l'une quelconque des revendications précédentes, comprenant le calcul d'un gradient d'une pente décrivant la relation entre le débit respiratoire maximal et la pression du sujet en mesurant ledit débit respiratoire maximal et la pression à deux résistances respiratoires différentes, puis en calculant ledit gradient.

7. Procédé selon l'une quelconque des revendications précédentes, comprenant l'estimation d'une puissance musculaire respiratoire maximale du sujet en multipliant la moitié de la puissance musculaire respiratoire maximale par la moitié du débit respiratoire maximal.

8. Procédé selon l'une quelconque des revendications précédentes, comprenant l'étape de calcul d'un facteur de puissance spécifique au sujet sur la base de la puissance musculaire respiratoire maximale moyenne d'une pluralité de respirations du sujet par rapport à une puissance musculaire respiratoire maximale du sujet et/ou

   comprenant l'étape de calcul du travail musculaire respiratoire accumulé en fonction du temps pour au moins une respiration du sujet, et/ou
   comprenant l'étape de calcul du travail musculaire respiratoire moyen pour une pluralité de respirations du sujet.

9. Procédé selon l'une quelconque des revendications précédentes, comprenant l'étape de calcul d'une rétroaction de travail spécifique au sujet d'au moins une respiration sur la base du travail musculaire respiratoire accumulé en fonction du temps par rapport à une capacité de travail musculaire respiratoire maximale pour une respiration du sujet ; et/ou comprenant l'étape de calcul d'une rétroaction de travail spécifique au sujet sur la base d'un travail musculaire respiratoire accumulé d'une pluralité de respirations du sujet par rapport à une capacité de travail musculaire respiratoire maximale de ladite pluralité de respirations.

10. Système de détection respiratoire pour un dispositif d'entraînement ou de test respiratoire ayant une voie aérienne d'inspiration et/ou d'expiration, le système de détection comprenant :

   - une unité de capteur électronique comprenant ;
   - au moins un capteur de pression pour mesurer la pression d'air dans la voie respiratoire d'inspiration et/ou la voie respiratoire d'expiration du dispositif d'entraînement et de test respiratoire, et
   - une unité de traitement pour transmettre des données de pression
   - un programme informatique comportant des instructions qui, lorsqu'elles sont exécutées par un dispositif de traitement à distance, tel qu'un smartphone (3), amènent le dispositif de traitement à :

      i. obtenir en continu lesdites données de pression via un récepteur sur le dispositif de traitement à distance, et
      ii. exécuter le procédé selon l'une quelconque des revendications précédentes.

11. Système de détection respiratoire selon la revendication 10, dans lequel le programme informatique comporte des instructions qui, lorsqu'elles sont exécutées par le dispositif de traitement à distance, amènent le dispositif de traitement à afficher des données de puissance et/ou de travail calculées instantanées et/ou cumulatives sur un écran du dispositif de traitement à distance.

12. Système d'entraînement et de test respiratoire pour exercer et analyser la respiration d'un sujet (1) comprenant :

   - une unité respiratoire (2) comprenant :

      • un embout buccal (18) relié à :

         - au moins une voie respiratoire d'inhalation ayant une résistance réglable au flux d'air d'inhalation (8),
         - au moins une voie respiratoire d'expiration ayant une résistance réglable au flux d'air d'expiration (9),

      • une unité de capteur électronique (7, 13) comprenant

         - au moins un capteur de pression pour mesurer la pression d'air dans l'embout buccal, et
         - une unité de traitement pour transmettre des données de pression
         - un programme informatique comportant des instructions qui, lorsqu'elles sont exécutées par un dispositif de traitement à distance, tel qu'un smartphone (3), amènent le dispositif de traitement à :

            - obtenir en continu lesdites données de pression via un récepteur sur le dispositif de traitement à distance, et
            - exécuter le procédé selon l'une quelconque des revendications précédentes 1 à 9.

13. Système d'entraînement et de test respiratoire selon la revendication 12, dans lequel le programme informatique comporte des instructions qui, lorsqu'elles sont exécutées par le dispositif de traitement à distance, amènent le dispositif de traitement à afficher des données de puissance et/ou de travail calculées instantanées et/ou cumulatives sur un écran du dispositif de traitement à distance.

14. Système de calcul de l'intensité d'entraînement respiratoire dans un ensemble de données représentant la respiration d'un sujet, comprenant un dispositif de stockage non transitif, lisible par ordinateur, pour stocker des instructions qui, lorsqu'elles sont exécutées par un processeur, exécutent un procédé de calcul de l'intensité d'entraînement respiratoire dans un ensemble de données représentant la respiration d'un sujet selon l'une quelconque des revendications 1 à 9.

15. Programme informatique comportant des instructions qui, lorsqu'elles sont exécutées par un dispositif ou un système informatique, amènent le dispositif ou le système informatique à calculer l'intensité d'entraînement respiratoire dans un ensemble de données représentant la respiration d'un sujet selon l'une quelconque des revendications 1 à 9.

FIG. 1

FIG. 2

FIG. 3

FIG. 4A

FIG. 4B

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4221381 A
- US 4739987 A
- DE 102019001926
- US 2009229611 A
- WO 2018011358 A

### Non-patent literature cited in the description

- *Respiratory device and system for exercising and analysing respiration of a subject*, 13 July 2017
- **MCCONNELL, A**. Respiratory Muscle Training: Theory and Practice. Elsevier, 2013